# EUROPEAN PATENT SPECIFICATION

(11) **EP 2 782 528 B1**
(45) Date of publication and mention of the grant of the patent: **30.09.2015**
(21) Application number: 12795597.9
(22) Date of filing: 21.11.2012
(51) Int. Cl.: A61F 2/46, A61F 2/34

(54) **SURGICAL INSTRUMENT HEAD AND ASSEMBLY INCLUDING TAB SEPARATION MEMBER**
KOPF FÜR EIN CHIRURGISCHES INSTRUMENT UND ANORDNUNG MIT EINEM LASCHENTRENNELEMENT
TÊTE D'INSTRUMENT CHIRURGICAL ET ENSEMBLE COMPRENANT UN ÉLÉMENT DE SÉPARATION DE LANGUETTE

(30) Priority: 23.11.2011 GB 201120199
(43) Date of publication of application: 01.10.2014
(73) Proprietor: DePuy (Ireland), Co Cork (IE)
(72) Inventor: TAYLOR, Andrew, Chilworth Southampton Hampshire SO16 7NS (GB); BIRD, Timothy, Chilworth Southampton Hampshire SO16 7NS (GB)
(74) Representative: Alton, Andrew
(86) International application number: PCT/GB2012/052881
(87) International publication number: WO 2013/076483

(56) References cited:
- EP-A2- 1 634 552
- WO-A1-2009/098491
- FR-A1- 2 897 528
- FR-A1- 2 917 288

## Description

The present invention relates in general to the field of orthopaedic medical implants and in particular to a medical implant assembly comprising a cup prosthesis with an attachment member and an instrument head attached to the attachment member.

Orthopaedic hip surgery is known. During such surgery, an implant may be used to line to the acetabulum of a patient's pelvis and provide a concave surface for articulation of the hip joint. Such an implant has a general form in the shape of a cup or bowl and is often referred to as a cup prosthesis or as an acetabular cup prosthesis.

A number of different forms have been proposed for a medical implant that can function as an acetabular cup prosthesis.

FR2917288 describes an acetabular cup having a greater than hemispherical shape and a tool for releasably gripping and implanting the cup. The tool has a gripper adapted to be secured to the cup, and includes a central part extending along a longitudinal axial direction. Two fixed anchorage fingers and an articulated, anchorage finger are arranged as a star around the central unit. The fingers are adapted to be anchored to formations in the outer surface of the cup using a bayonet attachment. The articulated finger has a hook, rotatably mounted along a rotational axle perpendicular to the longitudinal axis of the central unit. The tab is movable between a locking position in which the finger is anchored in the cup and a released position by translating an inner part of the tool, which acts on a free end of the articulated anchorage finger, along its longitudinal axis

Another example is discussed in EP-2174621 A1, which relates to a prosthesis comprising a ceramic acetabular cup and a metal band applied around the outer circumference of the cup adjacent to the rim of the cup. A ceramic acetabular cup has good wear characteristics and has good compressive strength. The metal band provides increased strength to the acetabular cup; in particular it increases the hoop compression on the cup prosthesis so that it is pre-stressed. This increases the strength of the prosthesis and reduces the likelihood of fracture when exposed to impaction forces during implantation.

WO-2010/146398 A1 discusses an acetabular cup prosthesis with a metal band applied around its circumference adjacent to the rim of the cup. In order to provide more secure attachment of the prosthesis to an impaction instrument, flanges extend from the metal band to connect to an impaction head.

In one construction of WO-2010/146398 A1, the flanges are elongated strips which are bent over the impaction head and secured close to the axial centre of the radial head. In another construction, one or more flanges extend upwardly from the metal band to engage an impaction head. In all the constructions, the flanges include a thinner region which is designed be frangible and broken once the prosthesis has been implanted, thereby releasing the instrument head from the prosthesis.

The thinner region is broken by rotating the instrument head relative to the prosthesis after implantation. The instrument head includes notches or lugs which apply pressure to the flanges or strips when the instrument head is rotated, causing the flanges to break at their weakest point, the position of the thinner region. In one construction, elongated strips are located within a notch that applies a torque to the elongated strip, breaking the elongated strip by shearing in the thinner region. In another construction, radially extending lugs on the instrument head are rotated into contact with a flange, applying an outward radial force to the flange, breaking by shearing in the thinner region.

A disadvantage of all these constructions is that the instrument head is rotated to break the elongated strips or flanges. Before the strips or flanges break, the torque resulting from this rotation may be transferred to the implanted prosthesis. This can cause the implanted prosthesis to move after implantation during removal of the instrument head. It would be desirable to minimise the forces acting on the implanted prosthesis during removal of the instrument head.

Accordingly, the present invention provides an instrument head comprising a tab separation member that can be rotated relative to the instrument head. The instrument head can then be held in place during rotation of the tab separation member to minimise the transfer of torque to an implanted prosthesis attached to the instrument head.

According to a first aspect of the invention, there is provided an assembly comprising: a cup prosthesis having a circumference and a central axis which defines an axial direction and a radial direction; an attachment member applied around the circumference of the cup, wherein the attachment member comprises: a circumferential band; and at least two tabs extending substantially in the axial direction from the circumferential band and having a circumferential region of weakness; and a surgical instrument head configured to engage the cup prosthesis and attach to the attachment member, the surgical instrument head comprising: a tab separation member rotatable about the central axis of the instrument head relative to the instrument head and having a formation for engaging a rotation instrument; and wherein the tab separation member defines at least one radial cam surface for engaging and breaking away the at least two tabs at the cirumferential area of weakness when the tab separation member is rotated in use.

The radial cam surface is a surface which varies in its radial distance from the central axis, so that rotation of the tab separation member can engage the attachment member with the cam surface and apply an outward radial force to the attachment member. In use, when the instrument head is attached to an attachment member, the outward radial force may be used to break a portion of the attachment member where the outward radial force is applied and separate the instrument head from the attachment member.

The formation for engaging a rotation instrument may have any suitable form, including but not limited to shapes with rotational symmetry such as regular polygons and stars. The formation may be a recess for engaging a corresponding protrusion on a rotation instrument or a protrusion for engaging a corresponding recess on a rotation instrument.

The central axis defines axial and radial directions, where the radial direction is analogous to the radial dimension r and the axial direction is analogous to the vertical direction z in cylindrical polar coordinates.

Preferably, the at least one cam surface is tapered in an axial direction such that the thickness of the cam surface in the axial direction is reduced towards a radially outward edge of the cam surface. This concentrates the outward force applied by the cam surface in use while allowing the tab separation member to have sufficient thickness and strength to apply the outward force without failing mechanically, for example by buckling or yielding. Preferably, the tapering is asymmetric and the radially outward edge of the cam surface is located on the side of the tab separation member which is closer to an attached implant in use. In that case surface of the tab separation member which is closer to an attached implant in use is preferably flat.

The tab separation member may comprise at least two cam surfaces. The at least two cam surfaces may be evenly distributed around the central axis (i.e. they are positioned at an angle of 360/*n* apart around the central axis, where n is the number of cam surfaces). This is advantageous when the attachment member comprises discrete tabs around its circumference which are broken when engaged by the cam surface.

Preferably the tab separation member is contained within the instrument head. This ensures that in use torque applied to the tab separation member is not transferred to a prosthesis attached to the instrument head.

In further embodiments, the instrument head may further comprise a rotation preventing element for engaging a face of the attachment member. This can prevent relative rotation between the instrument head and an attached implant, improving the effect of the tab separation member and further reducing the transfer of torque to an attached implant. The rotation preventing element may comprise a slot in the instrument head extending in an axial direction, one face of the slot may be a rotation preventing element. A rotation preventing element may also be provided which fits within a radial opening in the attachment member with a small tolerance and will therefore function as a rotation preventing element.

The instrument head may form part of an impaction or insertion instrument, or be configured for connection to an impaction or insertion instrument.

The or each radial cam surface can include a curved portion. A part or the whole of the cam surface can be curved.

The or each radial cam surface can include a straight portion. A part or the whole of the cam surface can be a straight portion. The or each radial cam surface can include a plurality of straight portions.

The tab separation member can be a plate. The plate can be annular. The radial cam surface or surfaces can extend from the plate.

The annular tab separation member can defines a central aperture. The central aperture can have a shape providing at least one formation engageable by a part of an instrument to impart rotational drive to the tab separation member. The shape can provide a plurality of formations.

The surgical instrument head can include an upper part and a lower part. The tab separation member can be located between the upper part and lower part.

The tab separation member can define a plurality of radial cam surfaces each for engaging a respective part of the attachment member in use

The surgical instrument head can further comprise an upper part bearing a formation arranged to be engaged by a part of an instrument for imparting rotational drive to the tab separation member. The formation can be shaped to prevent relative rotation between the surgical instrument head and the part of the tool. The formation can have a polygonal shape.

In a second aspect the invention provides a kit of parts comprising: an assembly according to the fisrt aspect of the invention and an instrument opearable to impart rotational drive to the tab separation member.

The instrument can comprise a housing having a first formation at a distal end which is engageable with a formation on an upper part of the surgical instrument head. The instrument can further comprise a member which is rotatable relative to the housing and having a second formation at a distal end which is engageable with a formation on the tab separation member.

Embodiments of the invention will now be described by way of example only and not limitation with reference to the accompanying drawings, in which:
Figure 1 depicts a perspective view of a surgical instrument head assembled with a cup prosthesis and attachment member according to a first embodiment of the invention;
Figure 2 depicts a top view of the assembly of Figure 1;
Figure 3 depicts a side view of the assembly of Figure 1;
Figure 4 depicts a cross-section along the line A-A in Figure 3;
Figure 5 depicts an exploded view of the assembly of Figure 1;
Figure 6 depicts a perspective view of a top portion of a surgical instrument head used in the assembly of Figure 1;
Figure 7 depicts a side view of the top portion of an instrument head depicted in Figure 6;
Figure 8 depicts a cross-section along line A-A in Figure 7;
Figure 9 depicts a perspective view of a bottom portion of a surgical instrument head used in the assembly of Figure 1;
Figure 10 depicts a side view of the bottom portion of the surgical instrument head depicted in Figure 9;
Figure 11 depicts a cross-section along line A-A in Figure 10;
Figure 12 depicts a perspective view of a tab separation member used in the assembly of Figure 1;
Figure 13 depicts a top view of the tab separation member depicted in Figure 12;
Figure 14 depicts a side view of the tab separation member depicted in Figure 12;
Figure 15 depicts a perspective view of an axial retaining element used in the assembly of Figure 1;
Figure 16 depicts a top view of the axial retaining element of Figure 15;
Figure 17 depicts a perspective view of an attachment member used in the assembly of Figure 1;
Figure 18 depicts a side view of the attachment member of Figure 17;
Figure 19 depicts a cross-section through line A-A in Figure 18;
Figure 20 depicts a perspective view of an assembly of a surgical instrument head, cup prosthesis and attachment member according to a second embodiment of the invention;
Figure 21 depicts a top view of the assembly of Figure 20;
Figure 22 depicts a side view of the assembly of Figure 20;
Figure 23 depicts a cross-section along line A-A in Figure 22;
Figure 24 depicts an exploded view of the assembly of Figure 20;
Figure 25 depicts a perspective view of a tab separation member used in the assembly of Figure 20;
Figure 26 depicts a top view of the tab separation member of Figure 25;

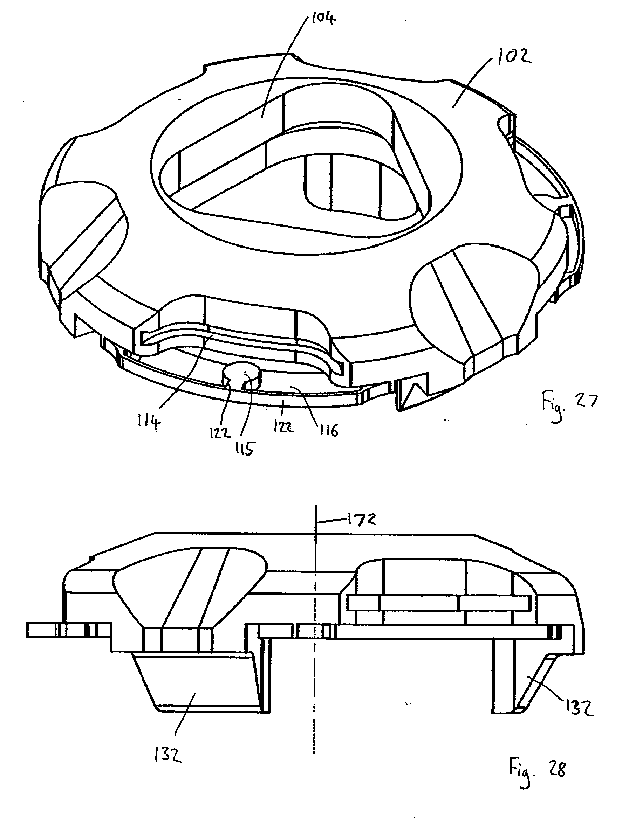

Figure 27 depicts a perspective view of a surgical instrument head used in the assembly of Figure 20;
Figure 28 depicts a side view of the surgical instrument head of Figure 27;
Figure 29 depicts a perspective view of an axial retaining element used in the assembly of Figure 20;
Figure 30 depicts a top view of the axial retaining element of Figure 29;
Figure 31 depicts a perspective view of a resilient element used in the assembly of Figure 20;
Figure 32 depicts a top view of the resilient element of Figure 31;
Figure 33 depicts a side view of the resilient element of Figure 31;
Figure 34 depicts a perspective view of an assembly of a surgical instrument head, cup prosthesis and attachment member according to a third embodiment of the invention;
Figure 35 depicts a top view of the assembly of Figure 34;
Figure 36 depicts a side view of the assembly of Figure 34;
Figure 37 depicts a cross-section along line A-A in Figure 36;

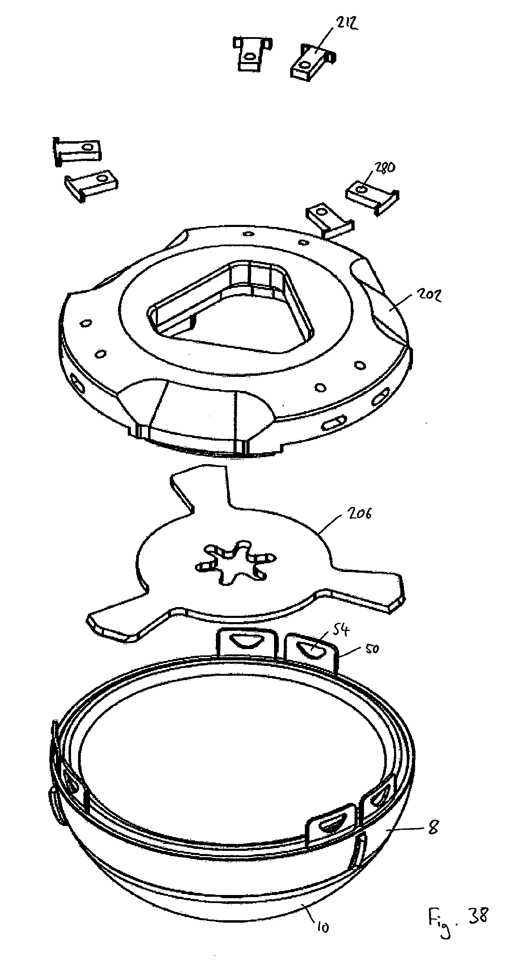

Figure 38 depicts an exploded view of the assembly of Figure 34;
Figure 39 depicts a perspective view of a surgical instrument head used in the embodiment of Figure 34;
Figure 40 depicts a side view of the surgical instrument head of Figure 39;
Figure 41 depicts a perspective view of a tab separation member used in the assembly of Figure 34;
Figure 42 depicts a top view of the tab separation member of Figure 40;
Figure 43 depicts a perspective view of a surgical instrument for rotating the tab separation member relative to the surgical instrument head;
Figure 44 depicts a top view of the surgical instrument of Figure 43;
Figure 45 depicts a cross-section along line A-A in Figure 43;
Figure 46 depicts a top view of the surgical instrument of Figure 43 and
Figures 47A and 47B are diagrammatic representations showing the shearing of tabs of an attachment element to disconnect a cup prosthesis from an attached surgical instrument head.

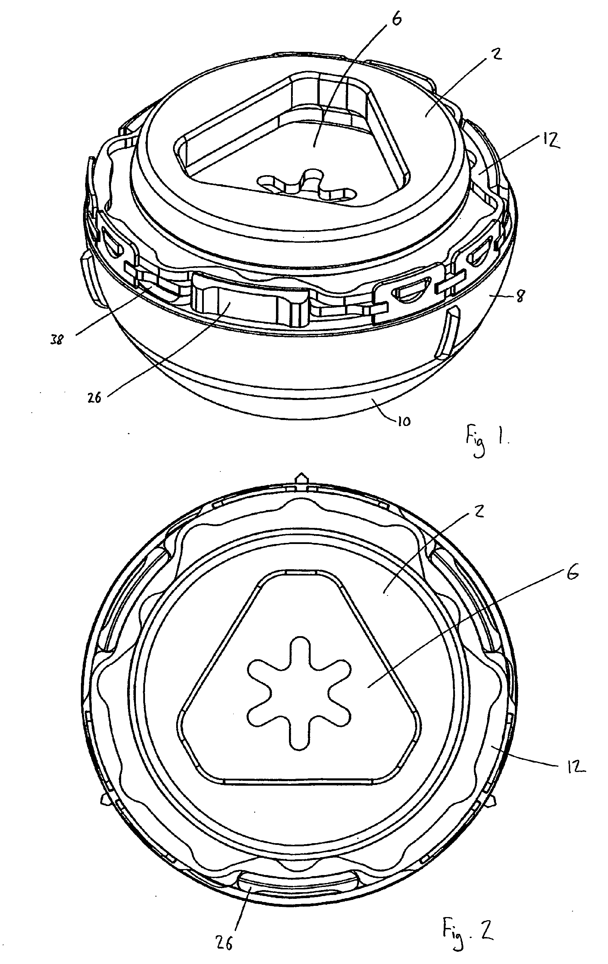

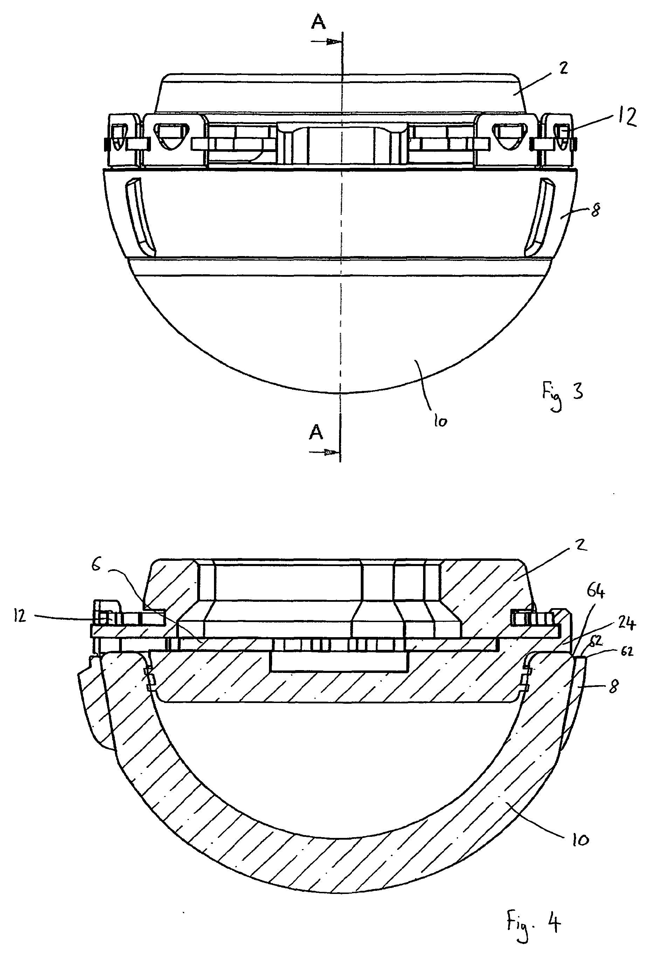

Figure 1 depicts a perspective view of an assembly of a surgical instrument head, cup prosthesis and attachment member according to a first embodiment of the present invention. Figure 2 depicts a top view of the assembly of Figure 1. Figure 3 depicts a side view of the assembly of Figure 1 and Figure 4 depicts a cross-section along line A-A in Figure 3. The assembly comprises a surgical instrument head, which comprises an upper part 2 and a lower part 24. A tab separation member 6 is contained within the surgical instrument head. An attachment member 8 is applied to a cup prosthesis 10. The surgical instrument head is engaged with the attachment member 8, and axially retained relative to the attachment member 8 using an axial retaining element 12.

As can best be appreciated with reference to the side view of Figure 3, the components of the assembly of Figure 1 are assembled coaxially, about a common axis defined by the cup prosthesis 10. This axis extends from the apex of the cup prosthesis 10 and defines an axial direction along the axis and a radial direction extending outwardly from the axis. The line A-A in Figure 3 follows the line of this central axis.

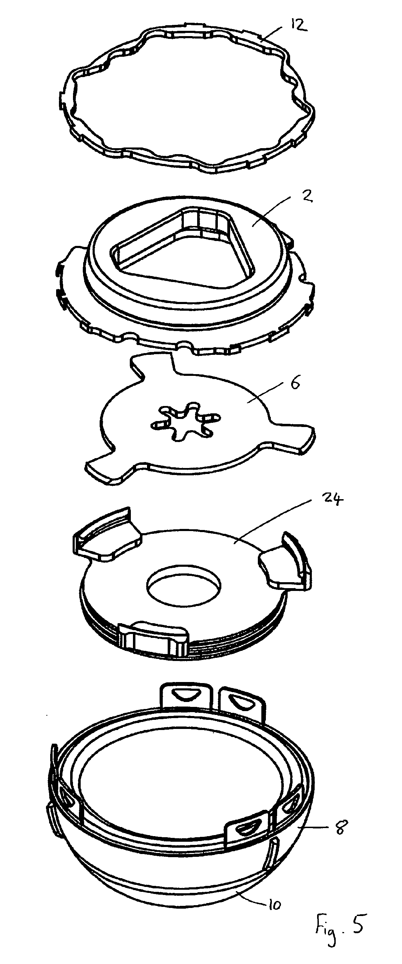

Figure 5 depicts an exploded view of the assembly of Figure 1. The construction of the individual components within the assembly of Figure 1 will now be described in more detail.

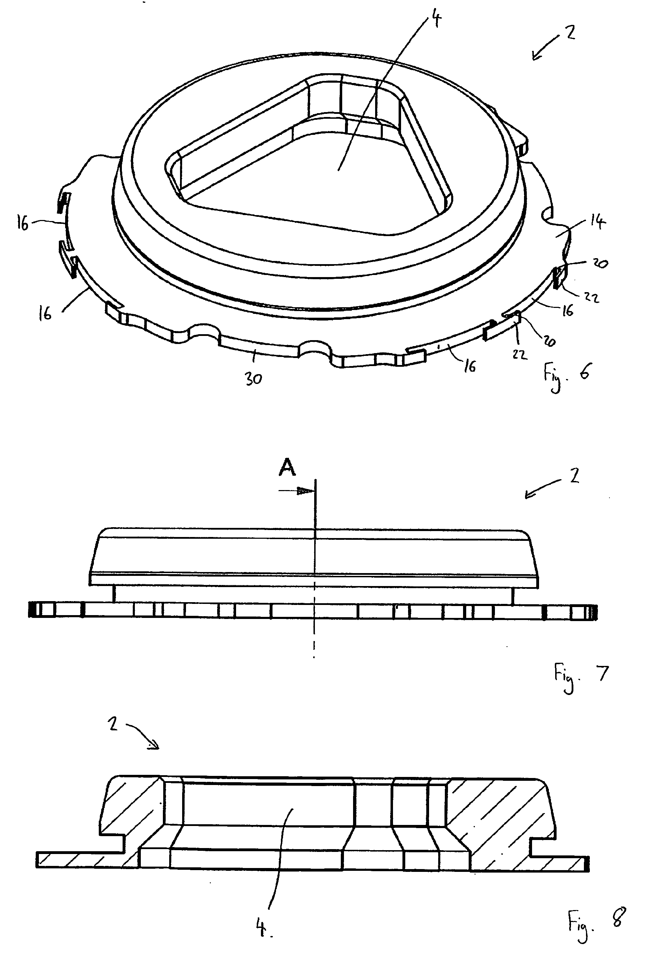

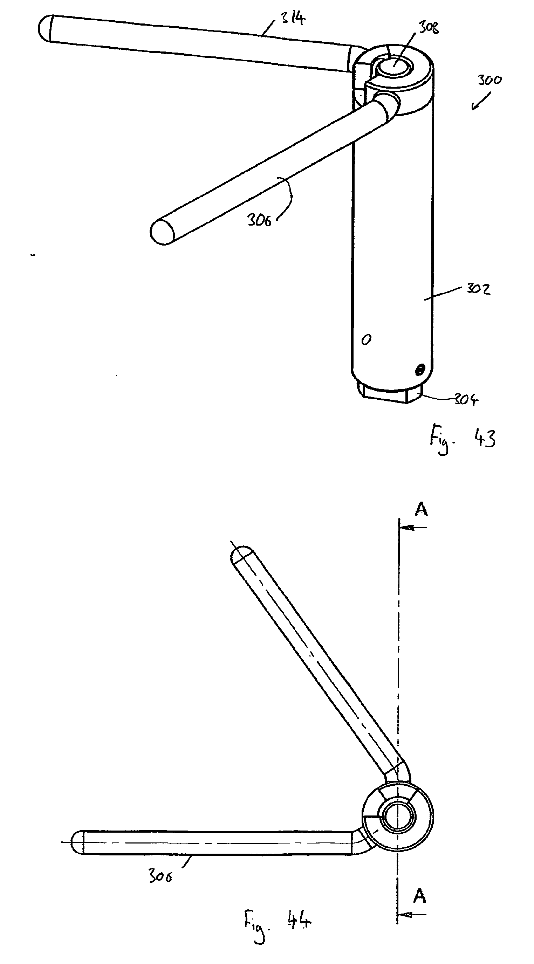

Figure 6 depicts a perspective view of a top part 2 of a surgical instrument head. The top part is preferably machined from a polymer, metal or metal alloy, for example 316 stainless steel. The top part 2 of the surgical instrument head defines an opening 4 which provides a generally triangular formation for engagement by a surgical instrument, allowing relative rotation of the surgical instrument head and the tab separation member 6. This surgical instrument will be described in more detail later, with reference to Figures 43 - 46. As can be seen in Figure 6 and in Figure 2, the formation is in the form of a truncated triangle which, when assembled is centred on the axis of the cup prosthesis 10.

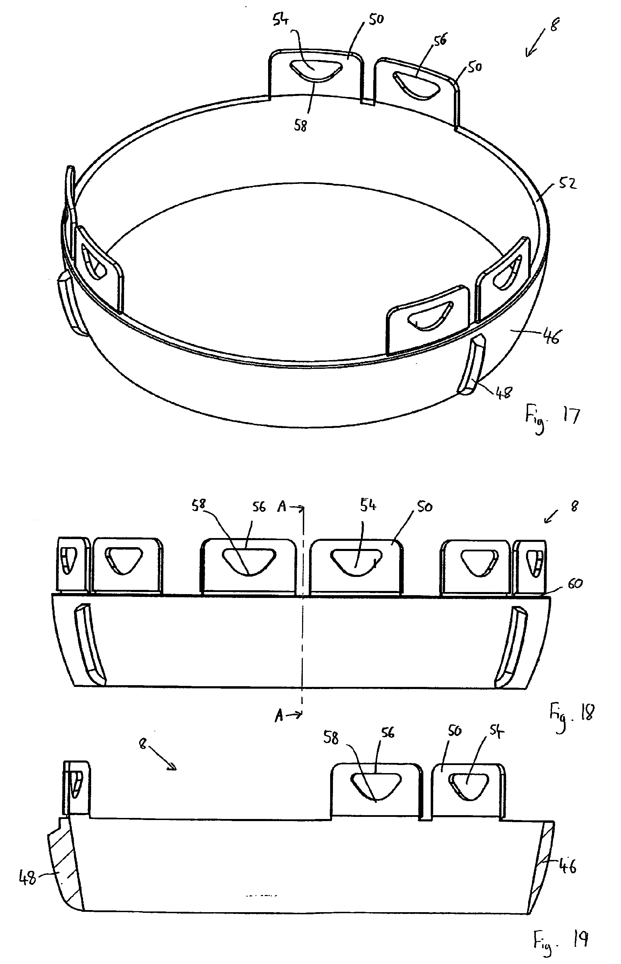

The top part 2 of the surgical instrument head also comprises a generally planar flange 14 which is parallel to the radial direction, perpendicular to the central axis. The flange 14 defines axially oriented slots 16 for receiving tabs of the attachment member 8 (the tabs of the attachment member 8 are described in more detail later with reference to Figures 17-19). The axially extending slots 16 include a face 20 which follows a line extending radially from the central axis. The face 20 can engage the side of a tab of the attachment member 8 to prevent relative rotation of the attachment member to the instrument head. The axially extending slots 16 also comprise a protrusion 22 which engages an outer circumferential face of the tab of the attachment member 8. These protrusions 22 function as a radial retaining element to prevent a tab from moving in the outward radial direction when the surgical instrument head is assembled and attached to an attachment member 8 of a cup prosthesis 10.

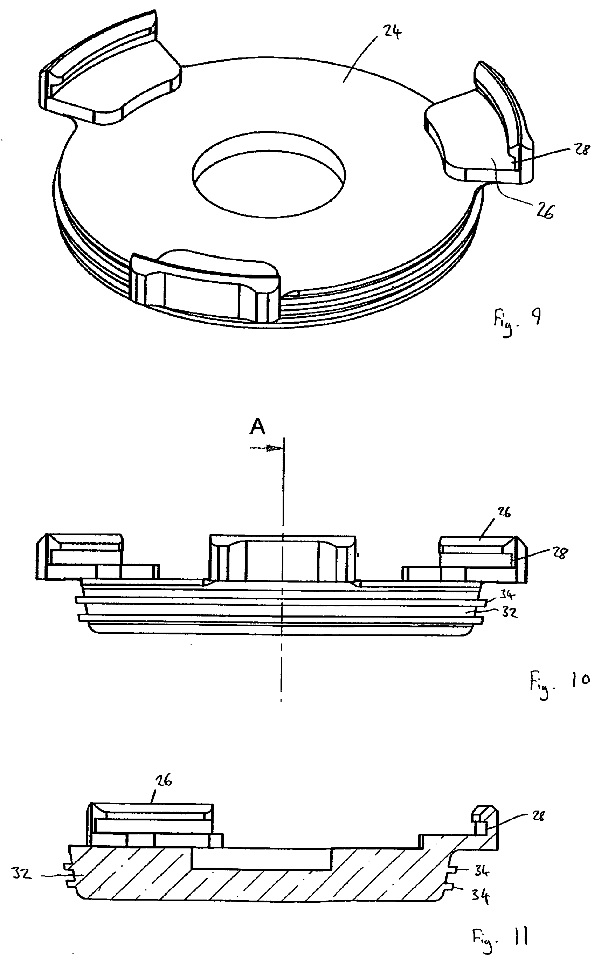

Other portions of the circumference of the flange 14 are indented in a radial direction relative to the axial slot 16. These engage discrete upwardly extending flanges 26 formed on a lower part 24 of the surgical instrument head which is depicted in perspective view in Figure 9. A side view of the lower part 24 is depicted in Figure 10, and a cross-section is depicted in Figure 11. As can be seen in Figure 9, the lower part 24 of the surgical instrument head comprises three flanges 26 which are radially spaced 120° apart about the central axis. These flanges 26 extend from an upper portion of the lower part 24. The flanges 26 define a partially circumferential slot 28 which follows a circumference centred on the central axis. This slot 28 receives the recessed portion 30 of the top part 2 of the surgical instrument head, to connect the top part 2 to the lower part 24 and prevent relative movement along the central axis and rotation about the central axis between the top part 2 and the lower part 24.

The lower part 24 also comprises a tapered member 32 extending below the flanges 26. This tapered member 32 extends continuously around a circumference centred on the central axis and tapers to a narrower diameter in the downward direction (towards the cup prosthesis when assembled). The tapered member 32 is provided with two circumferential ribs 34.

The diameter of the tapered member 32 is manufactured to be slightly less than the internal diameter of the cup prosthesis 10 (seen most clearly in Figure 4). However, the circumferential ribs 34 have a diameter which is larger than the internal diameter of the cup prosthesis 10. The ribs 34 are configured to deform plastically when the lower part 24 of the surgical instrument head is inserted into a cup prosthesis 10. This plastic deformation provides a tight fit, without requiring undue force. The continuous circumferential nature of the ribs provides a good seal around the entire circumference of the cup prosthesis 10.

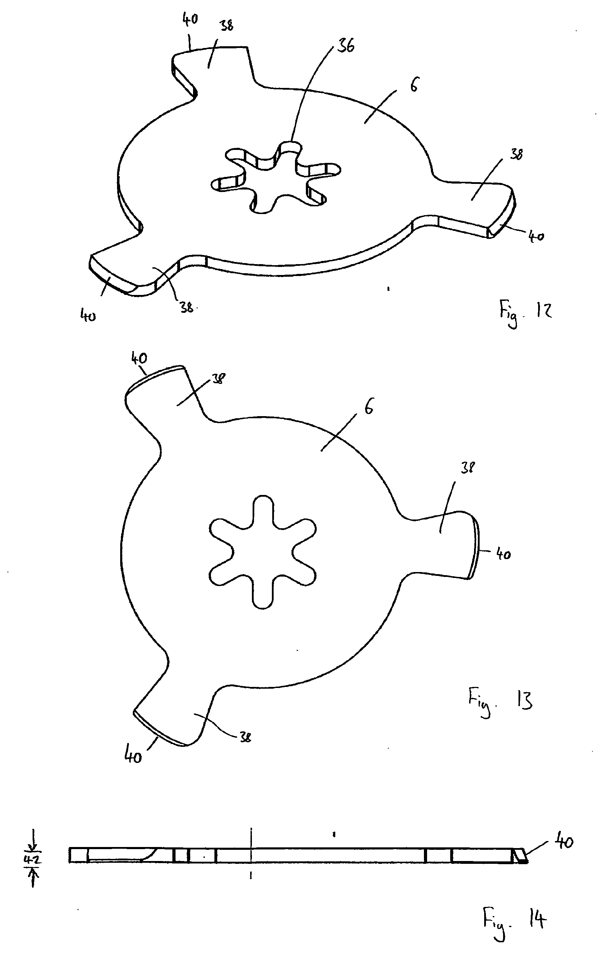

Referring now to Figure 12, a perspective view of a tab separation member 6 is depicted. A top view is shown in Figure 13 and a side view in Figure 14.

The tab separation member 6 comprises a formation 36 configured to engage a portion of a surgical instrument for relative rotation of the tab separation member 6 to the surgical instrument head. The formation 36 is generally star shaped and similar to the shape of the typographical character for an asterisk in this embodiment, although other shapes may also be used. The rotation instrument will be described later in more detail with reference to Figures 43 - 46.

The tab separation member 6 is provided with three protrusions 38 extending in a radial direction which each provide a cam surface 40 at their outer radial edge. The cam surface 40 is not concentric with the central axis of the tab separation member. The dimensions of the cam surface 40 are chosen such that the cam surface 40 is at a radial position slightly further from the central axis that the tabs of the attachment member 8 defined in more detail later.

The protrusions 38 are spaced 120° apart about the central axis and the tab separation member 6 has rotational symmetry about the central axis. As can be seen most clearly in Figures 12 and 14, the tab separation member 6 has a thickness 42 in the axial direction. This thickness 42 is required to give the tab separation member 6 sufficient mechanical strength. In order to concentrate the force applied by the cam edge 40 on a tab of the attachment member 8, the cam edge 40 is tapered in an upward direction, so that the radial dimension of the protrusion 38 is greater at a lower portion of the tab separation member 6 than an upper portion (the radial dimension is greater in the direction towards the cup prosthesis when assembled). Preferably, this edge is slightly rounded rather than sharp and therefore can be considered similar to a blunt knife edge.

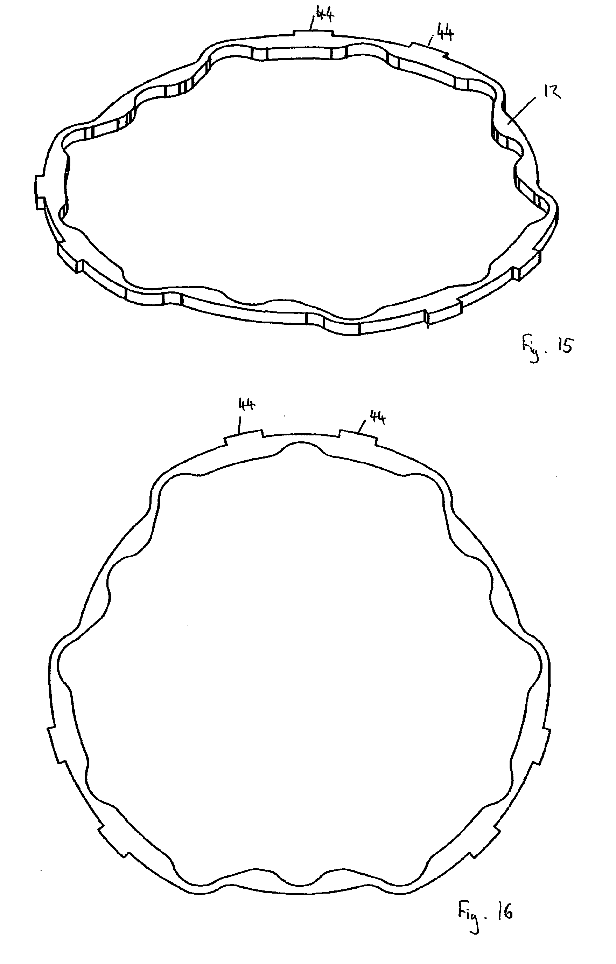

Referring to Figure 15, a perspective view of the axial retaining element 12 is depicted. Figure 16 depicts a top view of this axial retaining element 12. The axial retaining element 12 is received on top of the flange 14 of the top part 2 of the surgical instrument head. It is manufactured from a resilient material, for example a metal or metal alloy, preferably stainless steel, for example that sold under the trade name 17/4 PH and processed to Condition H900 commercially available from AK Steel, Ohio, USA.. As can be seen most clearly in Figure 16, the axial retaining element 12 has rotational symmetry about the central axis. It comprises protrusions 44 extending in a radial direction for engaging openings in the tab of the attachment member 8 (the attachment member 8 is described in more detail below).

The axial retaining element 12 has the form of a continuous, generally circumferential member which has a relatively thin radial thickness compared to its radius. This enables the axial retaining element 12 to be deformed radially inward if required. In the absence of an external force, the natural resilience of the material causes it to take the form depicted in Figures 15 and 16, with the radial protrusions 44 located about the central axis with rotational symmetry. In this embodiment, there are six radial protrusions 44 to engage the openings in six tabs of the attachment member 8. However, in other embodiments a different number of protrusions 44 may be provided, depending on the number of tabs in the attachment member 8.

Figure 17 depicts a perspective view of an attachment member 8. A side view is depicted in Figure 18 and a cross-section depicted in Figure 19.

The attachment member 8 comprises a circumferential band 46 which is applied around the circumference of the cup prosthesis 10. The circumferential band 46 comprises axial ribs 48 extending from it. Three axial ribs 48 are provided, spaced 120° apart about the central axis with rotational symmetry. In other embodiments, different numbers and arrangements of axial ribs 48 may be used.

In use, the circumferential band 46 remains in place attached to a cup prosthesis 10 once the cup prosthesis 10 has been implanted into a patient. The axial ribs 48 assist the implantation to the patient. In the cross-section of Figure 19, the left-hand side intersects one of the axial ribs 48.

The attachment member includes a plurality of tabs 50 which extend upwardly from an upper face 52 of the circumferential band 46. In this embodiment six tabs are provided spaced in groups of 2 at 120° intervals around the central axis. In other embodiments, different arrangement of tabs may be used.

The tabs 50 are adapted to be engaged by portions of the surgical instrument head and the axial retaining element 12 to attach the cup prosthesis 10 (to which the circumferential band 46 is attached) to the instrument head.

To facilitate this attachment, the tabs 50 each define an opening 54 which has a generally triangular shape. An upper surface 56 of the opening 54 is generally planar and parallel to a plane perpendicular to the central axis. This face 56 can therefore be engaged by a corresponding surface of the protrusion 44 of the axial retaining member 12 to prevent relative axial movement between the attachment member and the axial retaining member and therefore also prevent relative axial movement between the cup prosthesis 10 and the instrument head.

The opening 54 tapers downwardly (in the direction towards the cup prosthesis when assembled) with a generally triangular shape, leading to a rounded apex 58. This shape of opening 54 helps to distribute the forces exerted on the attachment member 8 during impaction, and initial surgical assessment of fixation of the cup prosthesis 10 into a patient. The symmetrical tapering and the rounded apex 58 distribute the forces evenly around the tab 50, avoiding stress concentrations and helping to avoid premature breakage of the tabs 50 from the circumferential band 46.

The tabs 50 are provided with a weakened circumferential region 60. In this embodiment, the tabs 50 are integrally formed with the circumferential band 48. A region of weakness 60 is provided by thinning the material of the tab in the portion adjacent to the upper face 52 of the circumferential band 46. This thinned area can be relatively easily broken to separate the tab 50 from the circumferential band 48 by the application of an outward radial force.

As can be seen most clearly in Figure 4, the upper face 52 of the circumferential band 46 has an outer circumferential edge 62, which is rounded to reduce potential trauma to the patient. The circumferential band 46 is mounted to the cup prosthesis 10 so that its upper face 52 is at an axial position which is lower than the outer edge 64 of the rim of the cup prosthesis 10. The outer edge 64 of the cup prosthesis 10 is also rounded. As can be seen in Figure 4, most clearly at the left-hand side, the rounded outer edge 62 of the upper face 52 and the rounded rim 64 of the cup prosthesis define a slight recess between them. The region of weakness 60 is positioned adjacent to the upper face 52 of the circumferential band 46 and therefore is located within this recess. Therefore, when the tab 50 is broken from the circumferential band 46, any rough edges remaining are located within the recess and shielded from a patient.

To assemble the embodiment of the present invention, the tab separation member 6 is sandwiched between the upper part 2 and the lower part 24 of the instrument head.

The attachment member 8 is attached to a cup prosthesis 10 by a thermal differential assembly process. It is preferred that the cup prosthesis 10 is manufactured from a ceramic or ceramic-like material and that the attachment member 8 is manufactured from metal or a metal alloy, for example titanium or a titanium alloy. In that case the attachment member can be press-fitted by heating the attachment member 8 using induction heating. The induction heating will not affect the ceramic cup prosthesis 10 and can be carried out by any suitable means. Typically, the attachment member 8 will be heated to about 350°C. Once fitted, the induction heating is removed and the attachment member will cool, shrinking and increasing the pre-stress applied to the cup prosthesis.

The attachment member 8 is slid into the assembled instrument head so that the tabs 50 align with the axial slots 16. The instrument head is then pressed into the cup prosthesis 10, so that the tapering member 32 functions as a locating member and is received against an inside face of the cup prosthesis 10. The deformable ribs 34 deform plastically against the surface of the cup prosthesis. This provides a tight fit and reduces the effect of tolerance stack up of the manufactured components. The ribs 34 align the central axis of the instrument head with the central axis of the cup prosthesis.

Finally, the protrusions 44 of the axial retaining element 12 are inserted into the openings or holes 54 of the tabs 50. Thus, the instrument head is securely attached to the cup prosthesis 10 with the attachment member 8. The tabs 50 are held securely so that they cannot move in axial or radial directions relative to the instrument head nor rotate about the central axis. The axial retaining element 12 prevents movement in an axial direction, the protrusions 22 of the axial slots 16 prevent movement in a radial direction and the faces 20, in addition to the fit of the protrusion 44 into hole 54, act to prevent rotation of the tab about the axis.

In use, it is desired to detach the tabs 50 from the circumferential band 46 along the region of weakness 60 after implantation. The circumferential band 46 then remains in place on the implanted cup prosthesis to provide additional strength while the tabs 50 are removed with the instrument head.

When initially assembled the tab separation member 6 is positioned so that the protrusions 38 are positioned at an angle about the longitudinal axis where they are not in contact with the tabs 50. In the absence of an applied torque, the protrusions 38 may move to a position where they are in contact with an edge of a tab 50, but they are prevented from moving further by friction between the protrusions 38 and the tab 50. Therefore, the protrusions 38 do not break the tabs 50 in the absence of an applied torque. Figure 1 depicts the protrusions 38 in the absence of an applied torque. As depicted in Figure 1, the protrusions 38 are in initial contact with the tabs 50 and are prevented from further movement towards the tabs in the absence of an applied toque by friction between the tabs 50 and protrusions 38.

In use, tabs 50 are broken by rotating the tab separation member 6 relative to the instrument head. This can be achieved using a surgical instrument as described later with reference to Figures 43 - 46. Rotation of the tab separation member engages the region of weakness with the cam edge 40. As the tab is restrained above the region of weakness by the protrusions 22, the tab cannot move outwardly in a radial direction in response to this force. Instead, the tab shears away from the circumferential band 46 in an outward direction. This detaches the tabs 50 from the circumferential band 46.

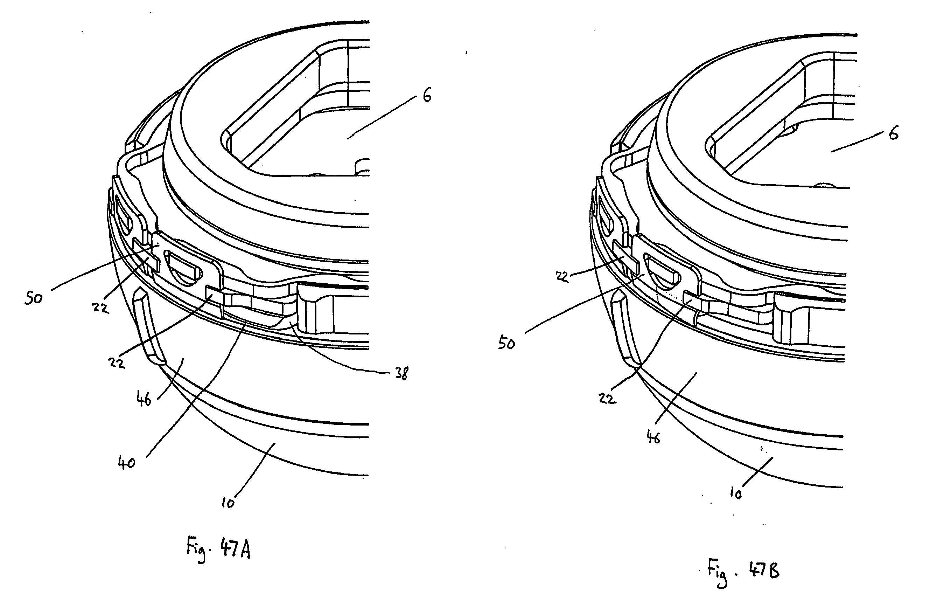

The operation of the tab separation member 6 to break the tabs 50 is shown in diagrammatic form in Figures 47A and 47B. For clarity, Figures 47A and 47A show a perspective view of the left half of an assembled cup prosthesis 10 and surgical instrument head, where one pair of tabs 50 can be seen. Figure 47A depicts the situation where the protrusion 38 of the tab separation member is initially in contact with the tab 50. In this position it is prevented from rotating in a clockwise direction by the engagement with the tab 50. When it is desired to disconnect the tabs 50, the tab separation member 6 is rotated clockwise relative to the cup prosthesis 10 and the surgical instrument head. This moves the protrusion 38 and cam edge 40 further into engagement with the tabs 50. The action of the cam edge 40 then pushes the region of weakness of the tab 50 in an outward radial direction. The tab 50 is restrained above the region of weakness by protrusions 22 of the instrument head. As a result, the tab 50 deforms outwardly at the region of weakness before breaking away from the circumferential band 46 by shearing at the region of weakness. This situation is depicted in Figure 47B. Figure 47B shows that the protrusion 38 and cam edge 40 has been rotated behind the tab 50. Tab 50 has begun deforming outward under the force applied by the cam edge and shearing away from the circumferential band at its right hand edge. The shape of the protrusion 38 and cam edge 40 in this embodiment results in the tab "peeling" away from the circumferential band 46 from its right hand edge. It will be appreciated that the same mechanism will act simultaneously on the three pairs of tabs in this embodiment because of the three protrusions 38 on the tab separation member. The action on a single pair of tabs is shown for clarity.

It is possible that sometimes a tab 50 may not completely detach from the circumferential band 46. In this situation, it is desirable to have a mechanism allowing manual detachment of the instrument head. In this case, manual detachment can be achieved by flexing the axial retaining member 12 in a radially inward direction, so that protrusions 44 disengage from holes 54. The instrument head can then be removed from any tabs remaining attached to the circumferential band by moving it in an axial direction. Any tabs remaining attached can then be broken away from the circumferential band by any other suitable means.

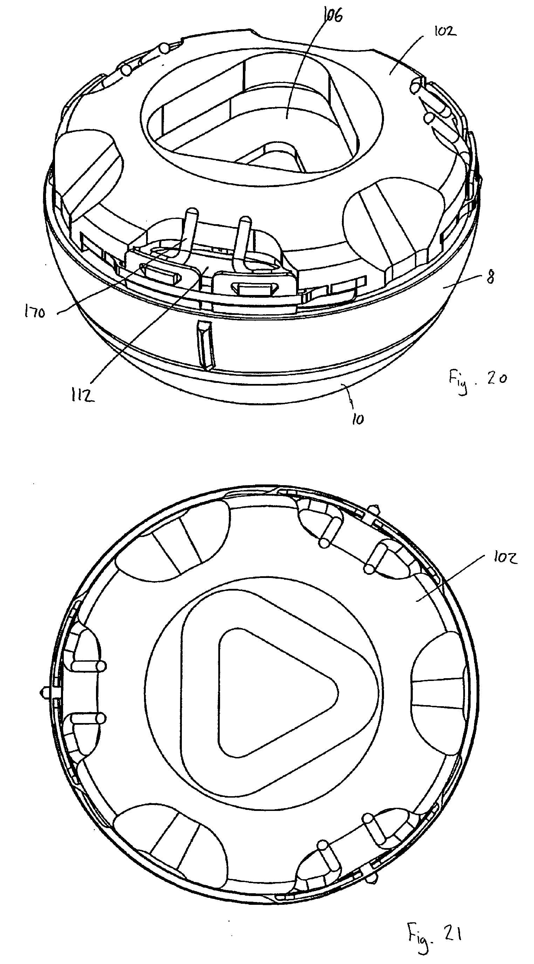

A second embodiment of an assembly of a surgical instrument head, cup prosthesis and attachment member is depicted in Figure 20. The construction of this embodiment is the same as the above-described first embodiment except as described below.

Figure 20 depicts a perspective view of an assembly comprising an instrument head 102, tab separation member 106, axial retaining element 112, resilient element 170, cup prosthesis 10 and attachment member 8.

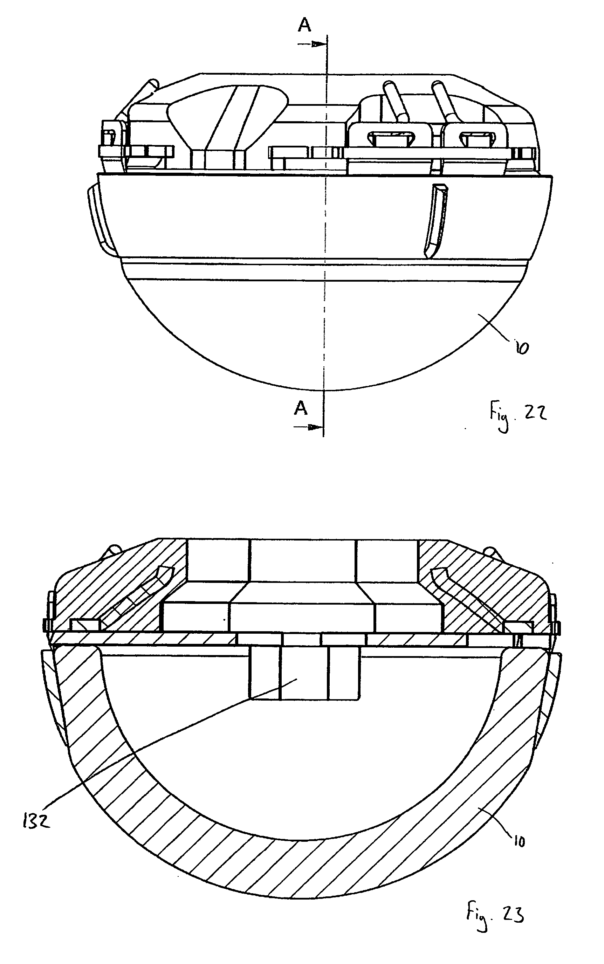

A top view of the assembly is show in Figure 21, Figure 22 depicts a side view and Figure 23 a cross-section along line A-A in Figure 22.

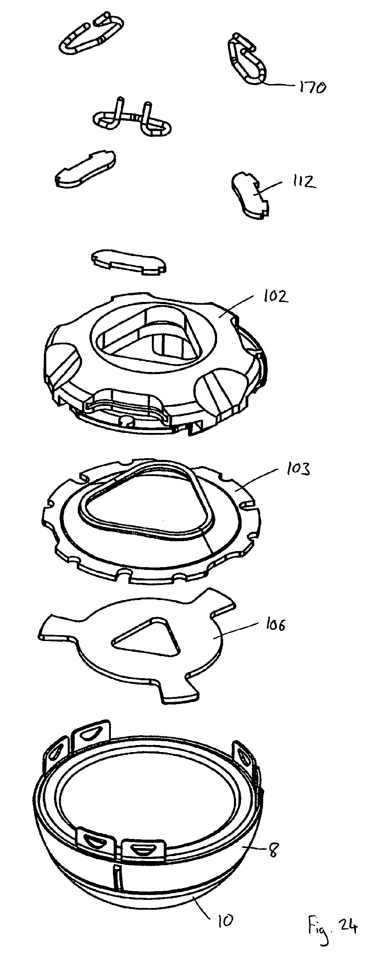

An exploded view of the assembly is depicted in Figure 24.

In this embodiment, the instrument head 102 is formed by injection moulding as a single piece. An upper portion of the surgical instrument head 102 defines an opening providing a formation 104 for engaging a rotation tool. The formation 104 is similar to the formation 4 described above with reference to Figure 6. However, in this embodiment, the formation 104 has a generally triangular shape with rounded corners of the triangle, so that it is more in the form of a rounded triangle than a truncated triangle.

A stainless steel plate 103 (visible in exploded view in Figure 24) is encapsulated within the injection moulded outer body that makes up the instrument head 102. The injection moulding takes place around the stainless steel plate 103. The stainless steel plate adds strength to the injection moulded plastic, allowing the component to withstand greater forces during implantation, and assessment of initial fixation.

The instrument head 102 defines axial openings 116 for receiving tabs from the attachment member 108. The slots 116 include an outer circumferential member 122 which engages an outer edge of the tab when assembled (seen most clearly in Figure 22). The axial slot 116 also include faces 120 which are parallel to the radial direction. In use, when a tab is received within slot 116, the member 122 prevents movement of the tab outwards in the radial direction and face 120 prevents relative rotation of the instrument head 102 relative to the tab about the central axis.

Instrument head 102 also defines a horizontal slot 114 which is parallel to a plane perpendicular to the central axis for receiving a retaining element 112 and a biasing element 170.

In order to ensure that the instrument head 102 is accurately aligned relative to the central axis 172, a plurality of locating members 132 extend downwardly from the instrument head 102 (in the direction towards the cup prosthesis when assembled). The locating members 132 are tapered in the direction away from the instrument head along the central axis, so that the external radial dimension of the locating element 132 is smaller in the direction towards the cup prosthesis. The locating elements 132 are arranged with rotational symmetry about the central axis 172. In this embodiment, three locating members 172 are provided which are spaced at 120° apart about the central axis 132. The taper angle of each of the locating members 132 is the same. The combination of these features enables the locating members 132 to accurately centre and align the central axis 172 of the instrument head 102 with the central axis of the cup prosthesis 110.

The instrument head 102 is preferably manufactured from a thermo setting polymer, for example nylon PA6. The locating members 132 are configured to deform plastically when the instrument head 102 is inserted into a cup prosthesis. This plastic deformation together with the rotational symmetry ensures that the instrument head achieves a tight, self-centred fit with the cup prosthesis so that its central axis is correctly aligned with the axis of the cup prosthesis 10.

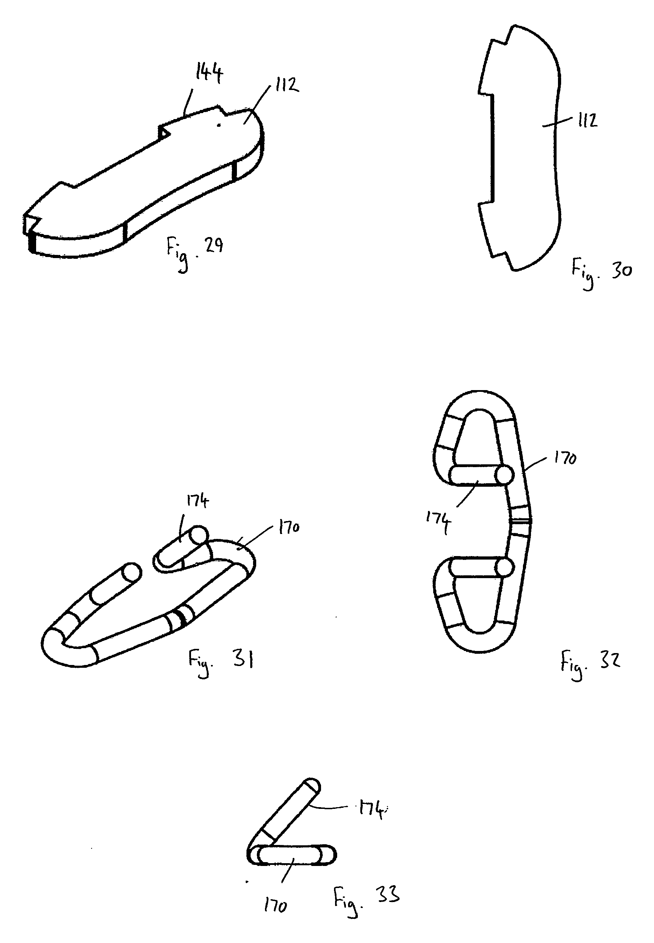

Referring to Figure 29, an axial retaining element 112 is depicted in perspective view. The top view is depicted in Figure 30. Unlike the single axial retaining element 12 described with reference to Figure 15, a plurality of discrete retaining elements are provided. As depicted in Figure 29, each retaining element includes two protrusions 144 for engaging holes formed in tabs of the attachment member. Three axial retaining elements 112 are provided, each received in a slot 114 of the instrument head and also supported by formation 115 extending inwardly from the radial retaining element 122. The axial retaining element 112 is biased in a radially outward direction by resilient element 170, depicted in perspective view in Figure 31, top view in Figure 32 and side view in Figure 33. Resilient element 170 is partially contained within slot 114 and includes free ends 174 which extend in an upward direction and resilient element 170 also engages the axial retaining element 112 to urge it outwardly.

The construction of the cup prosthesis 10 and attachment member 8 is the same as described above with reference to Figures 1-4 and 17-19.

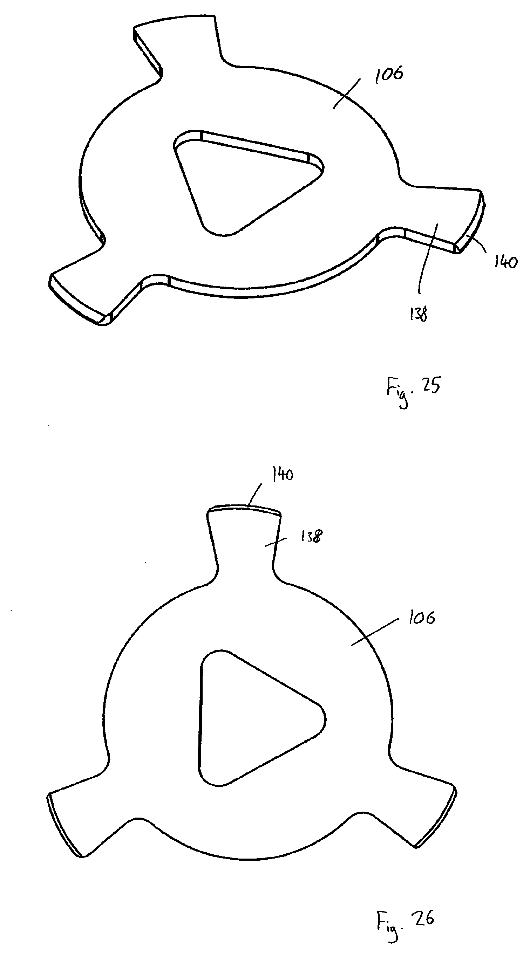

A tab separation member 106 is depicted in perspective view in Figure 25 and top view in Figure 26. This has generally the same construction as the tab separation member 6 described with reference to Figure 12, with three protrusions 138 each having a cam edge 140. The formation 136 for engaging a rotational tool is generally triangular in this embodiment.

The use and operation of this embodiment is generally as described above, with reference to Figures 1-19. The instrument head 102 is attached to the attachment member 8 by engaging the tabs 50 of the attachment member 8 with the axial slot 116 of the instrument head and engaging axial retaining element 112 with openings 54 of the tabs 50. When it is desired to disconnect the instrument head 102 from the attachment member 8, cam member 106 is rotated using a tool such as that described in Figures 43-46, to apply an outward force in the region of weakness of the tabs and break the tabs from the circumferential band.

Should the operation of the tab separation member 106 not work correctly, so that one or more of the tabs remain connected to the attachment member, this construction allows for manual disconnection of the instrument head by squeezing upwardly extending elements 174 of the resilient member 170 together. This deforms the member 170 in such a way that the outward force on the retaining element is removed, allowing the axial retaining element 112 to be slid radially inwards, disengaging protrusions 144 from openings 50.

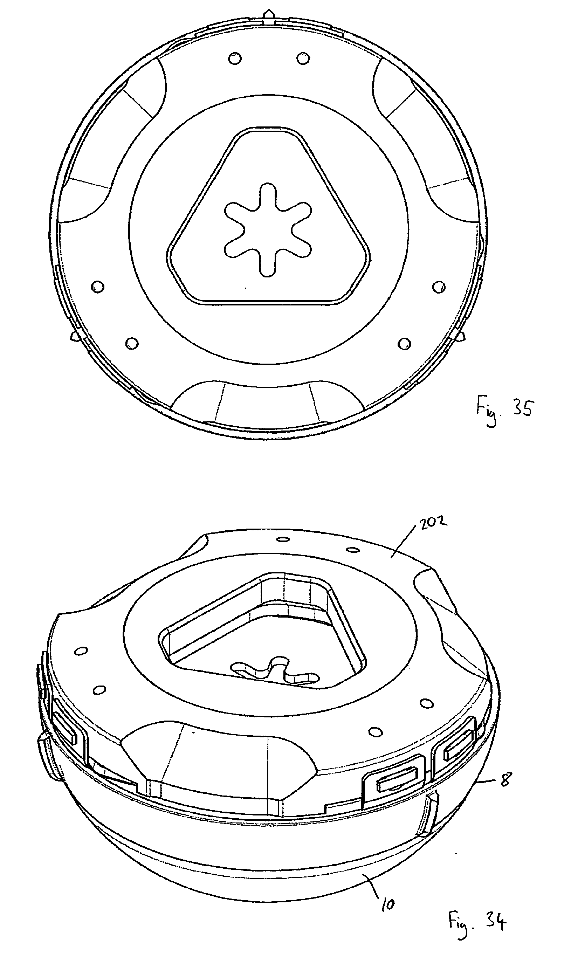

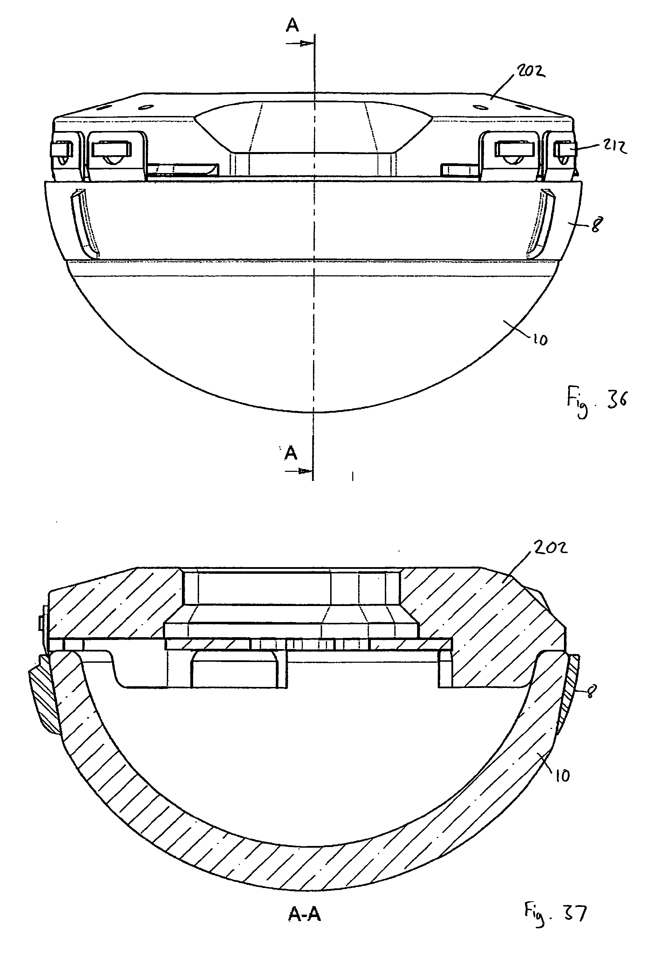

Figure 34 depicts a third embodiment of the present invention. A top view is depicted in Figure 35, a side view in Figure 36 and a cross-section in Figure 37. The construction of this embodiment is the same as the above-described first and second embodiments except as described below.

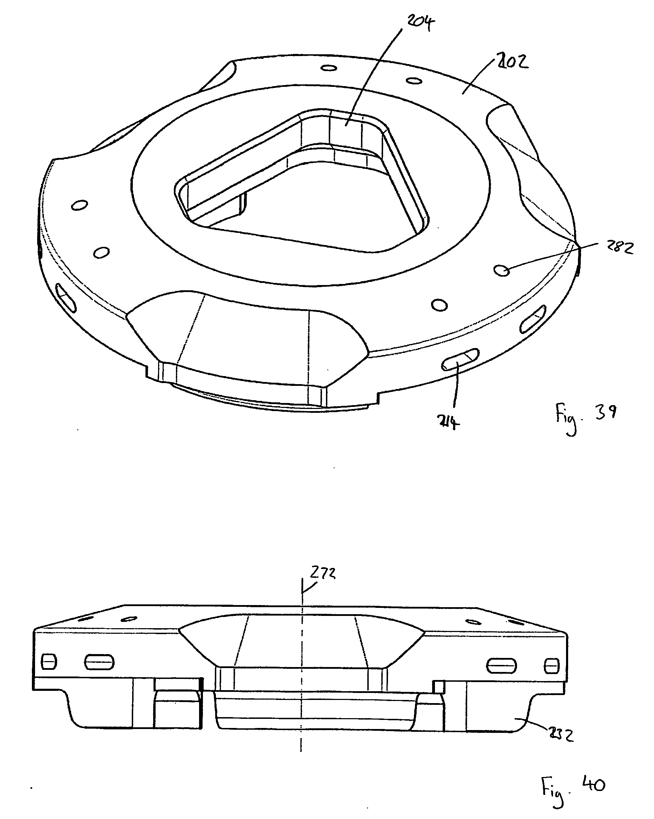

An instrument head 202 is formed as a single piece and is depicted in perspective view in Figure 39 and side view in Figure 40. Instrument head 202 includes horizontal slots 214 for receiving an axial retaining element 212. Instrument head 202 also defines an opening 204 having a formation for engaging a corresponding formation on a rotation instrument. The formation of the opening 204 is a truncated triangle, as described above with reference to Figure 6.

A stainless steel plate is preferably encapsulated within an injection moulded outer body that makes up the instrument head 202., in a similar way to the embodiment of Figure 24. The stainless steel plate adds strength to the injection moulded plastic, allowing the component to withstand greater forces during implantation, and assessment of initial fixation.

The surgical instrument head includes three tapered locating members 232 which extend axially downward from the surgical instrument head for engaging a cup prosthesis. The locating members 232 are preferably formed from nylon so that they deform plastically when the instrument head 202 is inserted into a cup prosthesis 10. The locating members 232 are provided with rotational symmetry about axis 272 so that a self-centering action is obtained to align axis 272 of the instrument head 202 with the axis of the cup prosthesis 10.

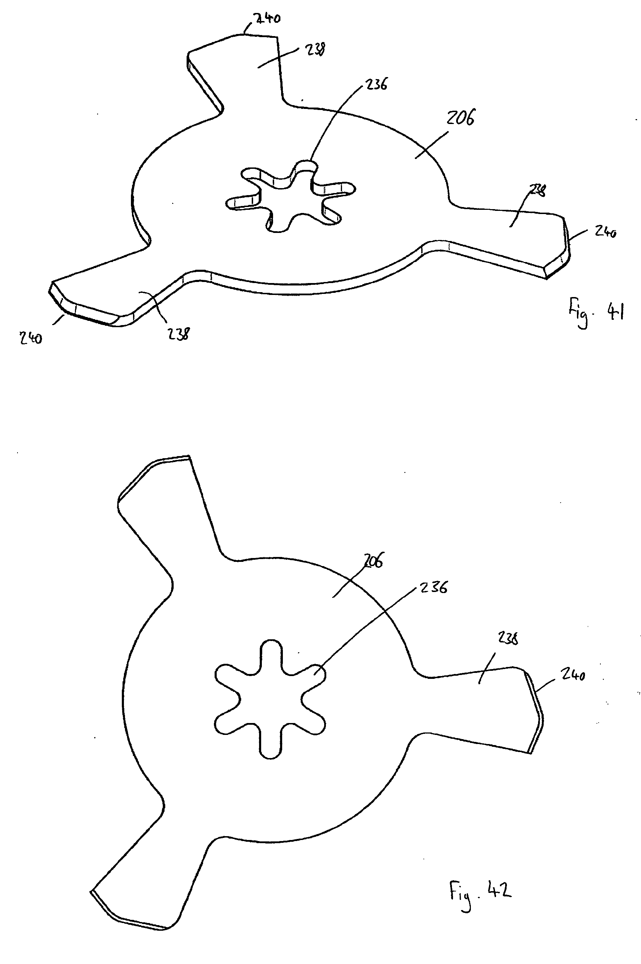

A tab separation member 206 is provided beneath the instrument head 202. The tab separation member 206 comprises three protrusions 238 defining cam surfaces 240 for engaging the weakened region of the tabs. A central opening 236 defines a generally star shaped formation for engaging a rotation instrument, such as the one described in more detail below with reference to Figures 43-46. The construction of tab separation member 206 is generally the same as the construction of tab separation member 6 described with reference to Figure 12. However, as can be seen by a comparison of Figures 41 and 42 with Figures 12 and 13, the profile of the cam edge 240 comprises two generally straight portions connected by a curve, rather than a single generally curved portion depicted with reference to Figures 12 and 13.

In use, the instrument head is assembled onto cup prosthesis 10 with attachment member 8 by placing it on the cup prosthesis so that slots 214 align with holes 54 in tabs 50. The retaining element 212 is then passed through hole 54 in tab 50 into slot 214 of the instrument head. The retaining element 212 includes an axial bore 280 which aligns with corresponding axial bores formed in the instrument head 282. The axial retaining element 212 can therefore be secured in the instrument head 202 by passing a rod or other cylindrical member through the bore 282 of the instrument head into the bore 212 of the radial retaining element 280.

In use, the tabs can be separated from the circumferential band by rotation of the tab separation member 206 relative to the instrument head 202. Should any of the tabs not be broken by this procedure, the tabs can be removed manually by removing the cylindrical member which retains the axial retaining elements 212 in the instrument head 202.

In this embodiment, the axial retaining element 280 also functions to prevent rotation of the instrument head relative to the attachment member, and as a radial retaining element preventing radial movement of the attachment member. Therefore, when the tabs are broken, the broken tabs remain securely attached to the instrument head 202.

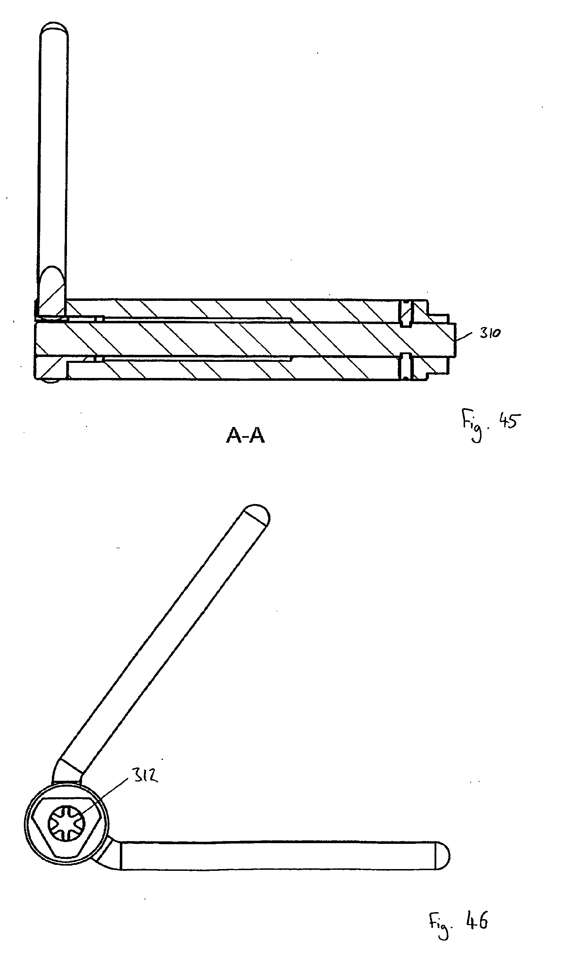

An embodiment of a surgical instrument 300, which can be used to activate the tab separation members described above and provide relative rotation of the tab separation member to the instrument head will now be described with reference to Figures 43-46. Figure 43 depicts a perspective view of the surgical instrument 300, Figure 44 a top view, Figure 45 a cross-section and Figure 46 a bottom view.

The surgical instrument comprises a first, generally tubular member 302 extending from a first engaging head 304 which has a formation of a truncated triangle, to engage the instrument head described above, with reference to the first and third embodiments. A rounded triangle engaging head (not shown) may be used with the second embodiment. A handle 306 extends in a radial direction from the first member 302.

A second member 308 is generally cylindrical and is provided within the tubular member 302, so that it is coaxial with member 302. A second engaging head 310 extends from the second member 308 and has a generally star shaped formation for engaging a corresponding formation provided on the tab separation member of the first and third embodiments. When used with the second embodiment, the second engaging head may have a generally triangular configuration (not shown). A second handle 314 extends from the second member 308.

In all the assemblies described above, rotation of the tab separation member through 360° is not required. The instrument 300 therefore limits the rotational position of the second member 308 relative to the first member 302. This is provided by locating the second handle 314 within a recess formed in the first member 300 which defines an arc of a circle. In this embodiment the arc is approximately 120°. This construction also allows handle 314 to be at the same axial position as handle 306, enhancing the ease of use.

In use, the first formation 304 is engaged with the instrument head and the second formation 312 is engaged with the tab separation member. Handle 306 is then held stationery while handle 314 is rotated relative to handle 306. This rotates formation 312 relative to formation 304, rotating the tab separation member relative to the instrument head so that the cam surface engages the weak region of the tabs and breaks the tabs from the circumferential band of the attachment member.

Although various embodiments have been described, it will be appreciated that the features of the above-described embodiments can be combined in other permutations than specifically as described. For example, different forms of tab separation members may be used in any of the embodiments. Various other modifications will suggest themselves to the person skilled in the art and are included within the scope of the invention, which is defined by the appended claims.

## Claims

1. An assembly comprising:
a cup prosthesis (10) having a circumference and a central axis which defines an axial direction and a radial direction;
an attachment member (8) applied around the circumference of the cup, wherein the attachment member comprises:
a circumferential band (46); and
at least two tabs (50) extending substantially in the axial direction from the circumferential band and having a circumferential region of weakness (60); and
a surgical instrument head configured to engage the cup prosthesis and attach to the attachment member, the surgical instrument head comprising:
a tab separation member (6, 106, 206) rotatable about the central axis of the instrument head relative to the instrument head and having a formation (36, 136, 236) for engaging a rotation instrument; and wherein the tab separation member defines at least one radial cam surface (40, 140, 240) for engaging and breaking away the at least two tabs at the cirumferential area of weakness when the tab separation member is rotated in use.

2. The assembly according to claim 1, wherein the at least one radial cam surface (40, 140, 240) is tapered in the axial direction such that the thickness of the cam surface in the axial direction is reduced towards a radially outward edge of the cam surface.

3. The assembly according to claim 2, wherein the tapering is asymmetric and the radially outward edge of the cam surface is located on the side of the tab separation member which is closer to the cup prosthesis.

4. The assembly according to any one of the preceding claims, wherein the tab separation member (6) is contained within the instrument head.

5. The assembly according to any one of the preceding claims, further comprising a rotation preventing element for engaging a face of the attachment member.

6. The assembly according to any preceding claim, wherein the radial cam surface (40, 140, 240) includes a curved portion.

7. The assembly according to any of claims 1 to 5, wherein the radial cam surface includes a straight portion.

8. The assembly according to any preceding claim, wherein the tab separation member (6, 106, 206) is an annular plate with the radial cam surface extending therefrom.

9. The assembly according to claim 8, wherein the annular plate defines a central aperture (36, 136, 236) having a shape providing at least one formation engageable by a part of an instrument to impart rotational drive to the tab separation member (6, 106, 206).

10. The assembly according to claim 9, wherein the shape provides a plurality of formations.

11. The assembly according to any preceding claim, wherein the surgical instrument head includes an upper part (2) and a lower part (24) and wherein the tab separation member (6) is located between the upper part and lower part.

12. The assembly according to any preceding claim, wherein the tab separation member (6, 106, 206) defines a plurality of radial cam surfaces (40, 140,240) each for engaging a respective tab (50) of the attachment member in use.

13. The assembly according to any preceding claim, wherein the surgical instrument head further comprises an upper part (2, 102, 202) bearing a formation arranged to be engaged by a part of an instrument for imparting rotational drive to the tab separation member and shaped to prevent relative rotation between the surgical instrument head and the part of the tool.

14. A kit of parts comprising:
the assembly according to any of claims 1 to 13; and
an instrument (300) opearable to impart rotational drive to the tab separation member.

15. The kit of parts of claim 14, wherein the instrument (300) comprises:
a housing (302) having a first formation (304) at a distal end which is engageable with a formation on an upper part of the surgical instrument head; and
a member (308) which is rotatable relative to the housing and having a second formation (312) at a distal end which is engageable with a formation on the tab separation member.

## Patentansprüche

1. Anordnung, umfassend:
eine Schalenprothese (10) mit einem Umfang und einer zentralen Achse, die eine axiale Richtung und eine radiale Richtung definiert;
ein Befestigungs-Element (8), das um den Umfang der Schale angebracht wird, wobei das Befestigungs-Element umfasst:
ein Umfangs-Band (46); und
zumindest zwei Anhänger (50), die sich im Wesentlichen in axialer Richtung von dem Umfangs-Band erstrecken und die eine umlaufende Schwäche-Region (60) aufweisen; und
ein Kopf eines orthopädischen Instruments, der dazu ausgebildet ist, mit der Schalen-Prothese zu koppeln und an dem Befestigungs-Element befestigt zu werden, wobei der Kopf des orthopädischen Instruments umfasst:
ein Anhänger-Trennungs-Element (6, 106, 206), das um die zentrale Achse des Instrumenten-Kopfs relativ zu dem Instrumenten-Kopf rotierbar ist und eine Anordnung (36, 136,236) zum Koppeln mit einem Rotations-Instrument aufweist; und wobei das Anhänger-Trennungs-Element zumindest eine radiale Nocken-Oberfläche (40, 140, 240) zum Koppeln und zum Abbrechen der zumindest zwei Anhänger an der umlaufenden Schwäche-Region, wenn das Anhänger-Trennungs-Element im Gebrauch gedreht wird, definiert.

2. Anordnung nach Anspruch 1, wobei die zumindest eine radiale Nocken-Oberfläche (40, 140, 240) in die axiale Richtung so verjüngt ist, dass die Dicke der Nocken-Oberfläche in die axiale Richtung zu einer radial außen liegenden Kante der Nocken-Oberfläche hin reduziert ist.

3. Anordnung nach Anspruch 2, wobei die Verjüngung asymmetrisch ist und die radial außen liegende Kante der Nocken-Oberfläche auf der Seite des Anhänger-Trennungs-Elements angeordnet ist, die näher zu der Schalen-Prothese ist.

4. Anordnung nach einem der vorherigen Ansprüche, wobei das Anhänger-Trennungs-Element (6) in dem Instrumenten-Kopf enthalten ist.

5. Anordnung nach einem der vorherigen Ansprüche, weiterhin umfassend ein Rotations-Verhinder-Element zum Koppeln mit einer Vorderseite des Befestigungs-Elements.

6. Anordnung nach einem der vorherigen Ansprüche, wobei die radiale Nocken-Oberfläche (40, 140, 240) einen gekrümmten Abschnitt umfasst.

7. Anordnung nach einem der Ansprüche 1 bis 5, wobei die radiale Nocken-Oberfläche einen geraden Abschnitt umfasst.

8. Anordnung nach einem der vorherigen Ansprüche, wobei das Anhänger-Trennungs-Element (6, 106, 206) eine ringförmige Platte ist, von der sich die radiale Nocken-Oberfläche erstreckt.

9. Anordnung nach Anspruch 8, wobei die ringförmige Platte eine zentrale Öffnung (36, 136, 236) definiert, die eine Form hat, die zumindest eine Anordnung bereitstellt, die mit einem Teil eines Instruments gekoppelt werden kann, um einen Rotations-Antrieb auf das Anhänger-Trennungs-Element (6, 106, 206) zu übertragen.

10. Anordnung nach Anspruch 9, wobei die Form eine Vielzahl von Anordnungen bereitstellt.

11. Anordnung nach einem der vorherigen Ansprüche, wobei der Kopf des orthopädischen Instruments einen oberen Teil (2) und einen unteren Teil (24) umfasst und wobei das Anhänger-Trennungs-Element (6) zwischen dem oberen Teil und dem unteren Teil angeordnet ist.

12. Anordnung nach einem der vorherigen Ansprüche, wobei das Anhänger-Trennungs-Element (6, 106, 206) eine Vielzahl von radialen Nocken-Oberflächen (40, 140, 240) definiert, wobei jede zum Koppeln mit einem jeweiligen Anhänger (50) des Befestigungs-Elements im Gebrauch geeignet ist.

13. Anordnung nach einem der vorherigen Ansprüche, wobei der Kopf des orthopädischen Instruments weiterhin einen oberen Teil (2, 102, 202) umfasst, der eine Anordnung trägt, die angeordnet ist, um mit einem Teil eines Instruments zum Übertragen eines Rotations-Antriebs auf das Anhänger-Trennungs-Element gekoppelt zu werden, und geformt um eine relative Rotation zwischen dem Kopf des orthopädischen Instruments und dem Teil des Werkzeugs zu verhindern.

14. Set von Bauteilen, umfassend:
die Anordnung nach einem der Ansprüche 1 bis 13; und
ein Instrument (300), das einsetzbar ist um einen Rotations-Antrieb auf das Anhänger-Trennungs-Element zu übertragen.

15. Set von Bauteilen nach Anspruch 14, wobei das Instrument (300) umfasst:
ein Gehäuse (302) mit einer ersten Anordnung (304) an einem distalen Ende, das koppelbar ist mit einer Anordnung auf einem oberen Teil des orthopädischen Instrumenten-Kopfs; und
ein Mitglied (308), das relativ zu dem Gehäuse rotierbar ist und an einem distalen Ende, das koppelbar ist mit einer Anordnung auf dem Anhänger-Trennungs-Element, eine zweite Anordnung (312) aufweist.

## Revendications

1. Ensemble comprenant :
une prothèse de cupule (10) ayant une circonférence et un axe central qui définit une direction axiale et une direction radiale ;
un élément de fixation (8) appliqué autour de la circonférence de la cupule, ou l'élément de fixation comprend :
une bande circonférentielle (46) ; et
au moins deux languettes (50) s'étendant sensiblement dans la direction axiale de la bande circonférentielle et ayant une région circonférentielle d'affaiblissement (60) ; et
une tête d'instrument chirurgical configurée pour venir en prise avec la prothèse de coupelle et pour être fixée à l'élément de fixation, la tête de l'instrument chirurgical comprenant :
un élément de séparation de languette (6, 106, 206) apte à tourner autour de l'axe central de la tête d'instrument relativement à la tête d'instrument et ayant une formation (36, 136, 236) pour la mise en prise avec un instrument de rotation ; et où l'élément de séparation des languettes définit au moins une surface de came radiale (40, 140, 240) pour la mise en prise avec et la rupture des au moins deux languettes à la zone d'affaiblissement circonférentielle lorsque l'élément de séparation des languettes est amené à tourner en cours d'utilisation.

2. Ensemble selon la revendication 1, dans lequel ladite au moins une surface de came radiale (40, 140, 240) est rétrécie dans la direction axiale de sorte que l'épaisseur de la surface de came dans la direction axiale est réduite vers un bord radialement extérieur de la surface de came.

3. Ensemble selon la revendication 2, dans lequel le rétrécissement est asymétrique, et le bord radialement extérieur de la surface de came se situe sur le côté de l'élément de séparation des languettes qui est plus proche de la prothèse de cupule.

4. Ensemble selon l'une quelconque des revendications précédentes, dans lequel l'élément de séparation des languettes (6) se trouve dans la tête d'instrument.

5. Ensemble selon l'une quelconque des revendications précédentes, comprenant en outre un élément de prévention de rotation pour la mise en prise avec une face de l'élément de fixation.

6. Ensemble selon l'une quelconque des revendications précédentes, où la surface de came radiale (40, 140, 240) comprend une portion courbée.

7. Ensemble selon l'une quelconque des revendications 1 à 5, dans lequel la surface de came radiale comprend une portion rectiligne.

8. Ensemble selon l'une quelconque des revendications précédentes, dans lequel l'élément de séparation des languettes (6, 106, 206) est une plaque annulaire avec la surface de came radiale s'étendant de celle-ci.

9. Ensemble selon la revendication 8, dans lequel la plaque annulaire définit une ouverture centrale (36, 136, 236) ayant une forme réalisant au moins une formation pouvant être mise en prise avec une partie d'un instrument pour impartir un entraînement rotationnel à l'élément de séparation des languettes (6, 106, 206).

10. Ensemble selon la revendication 9, dans lequel la forme réalise une pluralité de formations.

11. Ensemble selon l'une quelconque des revendications précédentes, où la tête de l'instrument chirurgical comprend une partie supérieure (2) et une partie inférieure (24) et où l'élément de séparation des languettes (6) se situe entre la partie supérieure et la partie inférieure.

12. Ensemble selon l'une quelconque des revendications précédentes, dans lequel l'élément de séparation des languettes (6, 106, 206) définit une pluralité de surfaces de came radiales (40, 140, 240) chacune pour la mise en prise avec une languette respective (50) de l'élément de fixation en cours d'utilisation.

13. Ensemble selon l'une quelconque des revendications précédentes, où la tête de l'instrument chirurgical comprend en outre une partie supérieure (2, 102, 202) portant une formation agencée pour une mise en prise avec une partie d'un instrument pour impartir un entraînement de rotation à l'élément de séparation des languettes et configurée pour prévenir une rotation relative entre la tête de l'instrument chirurgical et la partie de l'outil.

14. Kit de parties comprenant :
l'ensemble selon l'une quelconque des revendications 1 à 13 ; et
un instrument (300) apte à impartir un entraînement de rotation à l'élément de séparation des languettes.

15. Kit de parties selon la revendication 14, dans lequel l'instrument (300) comprend :
un boîtier (302) ayant une première formation (304) à une extrémité distale qui peut être mise en prise avec une formation sur une partie supérieure de la tête de l'instrument chirurgical ; et
un élément (308) qui peut tourner relativement au boîtier et ayant une deuxième formation (312) à une extrémité distale qui peut être mise en prise avec une formation sur l'élément de séparation des languettes.
